(19) Europäisches Patentamt / European Patent Office / Office européen des brevets

(11) **EP 4 394 026 A1**

(12) **EUROPEAN PATENT APPLICATION**
published in accordance with Art. 153(4) EPC

(43) Date of publication:
**03.07.2024  Bulletin 2024/27**

(21) Application number: **22861435.0**

(22) Date of filing: **25.08.2022**

(51) International Patent Classification (IPC):
*C12M 1/00* (2006.01)     *C12N 1/00* (2006.01)
*B32B 7/02* (2019.01)     *B32B 7/12* (2006.01)

(52) Cooperative Patent Classification (CPC):
**B32B 7/02; B32B 7/12; C12M 1/00; C12N 1/00**

(86) International application number:
**PCT/JP2022/032050**

(87) International publication number:
**WO 2023/027147 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **26.08.2021   JP 2021138142**

(71) Applicant: **TOKYO OHKA KOGYO CO., LTD.
Kawasaki-shi, Kanagawa 211 0012 (JP)**

(72) Inventors:
• **YOSHIOKA, Takahiro
  Kawasaki-shi, Kanagawa 211-0012 (JP)**

• **FUJIMOTO, Takashi
  Kawasaki-shi, Kanagawa 211-0012 (JP)**
• **KAMIZONO, Takashi
  Kawasaki-shi, Kanagawa 211-0012 (JP)**
• **AKABANE, Takafumi
  Tokyo 104-0032 (JP)**
• **SATOH, Taku
  Tokyo 104-0032 (JP)**
• **MORIGUCHI, Hiroyuki
  Tokyo 104-0032 (JP)**

(74) Representative: **Plasseraud IP
66, rue de la Chaussée d'Antin
75440 Paris Cedex 09 (FR)**

(54) **CHIP FOR CELL CULTURING, DEVICE FOR CELL CULTURING, METHOD FOR MANUFACTURING CHIP FOR CELL CULTURING, AND METHOD FOR CULTURING CELLS**

(57)     A chip for cell culture, including a laminate having a flow path structure inside, in which the laminate includes a bottom plate substrate, a flow path substrate in which a flow path is formed by laser processing, and a top plate substrate in this order, and the flow path substrate includes a resin having a glass transition temperature of 37°C or higher, an elastic modulus at 25°C of $1 \times 10^9$ Pa or more, and an elastic modulus at 150°C of $1 \times 10^7$ Pa or less.

FIG. 3

## Description

[Technical Field]

The present invention relates to a chip for cell culture, a device for cell culture, a method for producing a chip for cell culture, and a method for culturing cells.
Priority is claimed on Japanese Patent Application No. 2021-138142, filed August 26, 2021, the content of which is incorporated herein by reference.

[Background Art]

In monolayer culture which has been used for cell assays in the related art, an environment surrounding the cells is greatly different from that in vivo. Therefore, there often occurs a problem in that in cultured cells in the monolayer culture, most of functions expressed by cells in vivo are lost. It is expected that the problem is solved by advances in microfabrication technology and three-dimensional culture technology in recent years so that cell assays are improved in terms of throughput and reliability at the same time. In particular, the concept of organ-on-a-chip, which handles a chip for cell culture provided with a microfluidic device that reproduces a physiological three-dimensional culture environment in vitro as one organ, has spread, and a research in consideration of the applications for drug development has been widely developed worldwide. Furthermore, the concept of a body-on-a-chip, which aims to reproduce individual responses by connecting a plurality of organ models reconstructed in vitro with microflow paths and the like, has been proposed and has been attracting attention rapidly. Such a system is also referred to as a microphysiolocical system (MPS).
As the chip for cell culture as described above, there has been proposed a chip for cell culture, provided with a microflow path structure body having a hollow structure, where a substrate in which a flow path for cell culture is formed and a top plate blocking the flow path for cell culture are thermocompression-bonded through an adhesive layer which is a polyester-based resin having a Tg of 5°C or higher (see, for example, Patent Document 1).

[Citation List]

[Patent Document]

[Patent Document 1]
Japanese Unexamined Patent Application, First Publication No. 2018-102236

[Summary of Invention]

[Technical Problem]

The human mimicry systems as described above have attracted attention as a method for evaluating the efficacy and the safety of a drug in vitro. In evaluation of the drug, cells are cultured in the presence of a drug, and an effect of the drug on the cell is observed in a chip for cell culture constituting an MPS.
As the chip for cell culture used for the evaluation of the drug, there is a demand for conditions under which there is no leakage of a cell medium under a perfusion condition of around 37°C close to the human body temperature; culture and observation of cells to be evaluated are possible; and there is a less reduction in the drug. In order to satisfy the condition of a less reduction in the drug, it is necessary to suppress the adsorption of the drug onto a partition wall and the like forming a flow path during the perfusion of a drug solution. However, in a case where a flow path is formed by laser processing, microscopic irregularities are generated on a processed surface. The drug is easily adsorbed onto these irregularities, which causes a reduction in the drug in the drug solution.
The present invention has been made in view of the circumstances, and an object thereof is to provide a chip for cell culture, which can suppress adsorption of a drug onto a laser-processed flow path partition wall; a device for cell culture, including the chip for cell culture; a method for producing the chip for cell culture; and a method for culturing cells, using the chip for cell culture.

[Solution to Problem]

In order to accomplish the object, the present invention adopted the following configurations.
A first aspect of the present invention is a chip for cell culture, including a laminate having a flow path structure inside, in which the laminate includes a bottom plate substrate, a flow path substrate in which a flow path is formed by laser processing, and a top plate substrate in this order, and the flow path substrate includes a resin having a glass transition temperature of 37°C or higher, an elastic modulus at 25°C of $1 \times 10^9$ Pa or more, and an elastic modulus at 150°C of $1 \times 10^7$ Pa or less.

A second aspect of the present invention is a device for cell culture, including the chip for cell culture of the first aspect.

A third aspect of the present invention is a method for producing a chip for cell culture, including a laminate having a flow path structure inside, the method including a step A of laminating a bottom plate substrate, a flow path substrate in which a flow path is formed by laser processing, and a top plate substrate in this order; and a step B of bonding the respective laminated substrates, in which the flow path substrate includes a resin having a glass transition temperature of 37°C or higher, an elastic modulus at 25°C of $1 \times 10^9$ Pa or more, and an elastic modulus at 150°C of $1 \times 10^7$ Pa or less.

A fourth aspect of the present invention is a method for culturing cells, including a step of culturing cells in the presence of a drug within the flow path of the chip for cell culture of the first aspect.

[Advantageous Effects of Invention]

According to the present invention, there are provided a chip for cell culture, which can suppress adsorption of a drug onto a laser-processed flow path partition wall; a device for cell culture, including the chip for cells; a method for producing the chip for cell culture; and a method for culturing cells, using the chip for cell culture.

[Brief Description of Drawings]

FIG. 1 is a perspective view of a chip for cell culture according to an embodiment.
FIG. 2 is a dissembling view of the chip for cell culture of FIG. 1.
FIG. 3 is a cross-sectional view of the chip for cell culture of FIG. 1, taken along a cut line III-III.
FIG. 4 is a cross-sectional view of the chip for cell culture of FIG. 1, taken along a cut line IV-IV.
FIG. 5 shows an example of a layer configuration of the chip for cell culture according to an embodiment.
FIG. 6 shows an example of a layer configuration of the chip for cell culture according to an embodiment.
FIG. 7 is a schematic view showing an example of cell culture using the chip for cell culture according to an embodiment.

[Description of Embodiments]

Hereinafter, embodiments of the present invention will be described in detail with reference to the drawings depending on cases. In the drawings, the same or corresponding portions will be denoted by the same or corresponding reference numerals, and overlapping descriptions will be omitted. Dimensional ratios in each drawing are exaggerated for explanation and do not necessarily match the actual dimensional ratios.

(Chip for Cell Culture)

A first aspect of the present invention is a chip for cell culture, including a laminate having a flow path structure inside. The laminate includes a bottom plate substrate, a flow path substrate in which a flow path is formed by laser processing, and a top plate substrate in this order. The flow path substrate includes a resin having a glass transition temperature of 37°C or higher, an elastic modulus at 25°C of $1 \times 10^9$ Pa or more, and an elastic modulus at 150°C of $1 \times 10^7$ Pa or less.

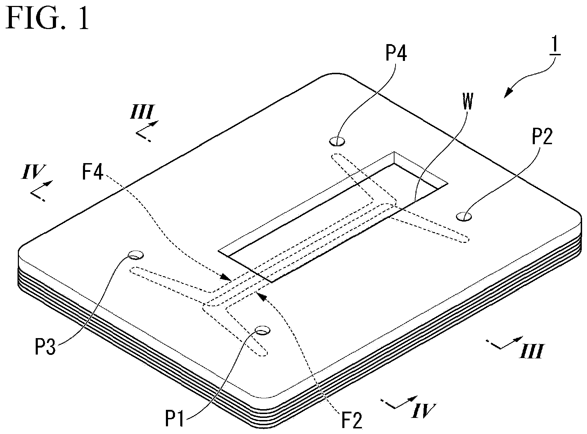

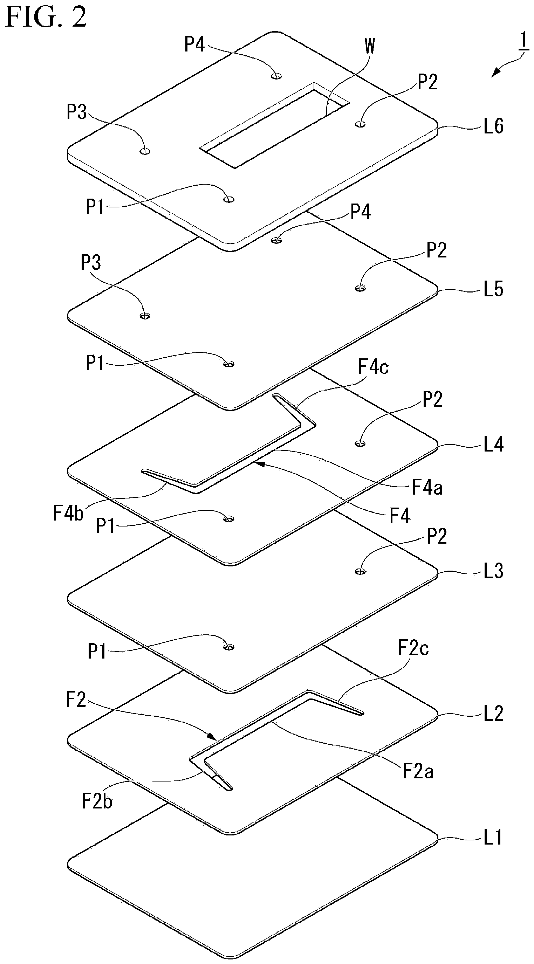

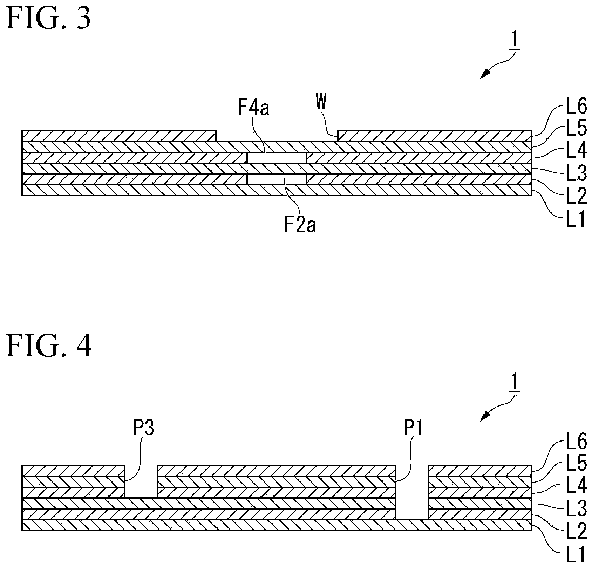

FIG. 1 is a perspective view of a chip for cell culture according to an embodiment. FIG. 2 is a dissembling view of a chip 1 for cell culture of FIG. 1. FIG. 3 is a cross-sectional view of the chip 1 for cell culture of FIG. 1, taken along a cut line III-III. FIG. 4 is a cross-sectional view of the chip 1 for cell culture of FIG. 1, taken along a cut line IV-IV.

The chip 1 for cell culture is a laminate having a flow path structure inside. The laminate of the chip 1 for cell culture is composed of a bottom plate substrate L1, a first flow path substrate L2, a porous membrane L3, a second flow path substrate L4, a top plate substrate L5, and a cover substrate L6. The bottom plate substrate L1, the first flow path substrate L2, the porous membrane L3, the second flow path substrate L4, the top plate substrate L5, and the cover substrate L6 are laminated in this order.

A first flow path F2 is formed in the first flow path substrate L2. A second flow path F4 is formed in the second flow path substrate L4. By laminating the respective substrates as described above, a flow path structure consisting of the first flow path F2 and the second flow path F4 is formed inside the chip 1 for cell culture.

The chip 1 for cell culture is provided with a port P1 as a drug solution introduction port into the first flow path F2, a port P2 as a drug solution discharge port from the first flow path F2, a port P3 as a drug solution introduction port into the second flow path F4, and a port P4 as a drug solution discharge port from the second flow path F4.

The first flow path F2 is formed of a first central flow path F2a, a first introduction flow path F2b that connects the port P1 and the first central flow path F2a, and a first discharge flow path F2c that connects the port P2 and the first central flow path F2a. A drug solution introduced from the port P1 is discharged from the port P2 through the first introduction flow path F2b, the first central flow path F2a, and the first discharge flow path F2c.

The second flow path F4 is formed of a second central flow path F4a, a second introduction flow path F4b that connects the port P3 and the second central flow path F4a, and a second discharge flow path F4c that connects the port P4 and the second central flow path F4a. A drug solution introduced from the port P3 is discharged from the port P4 through the second introduction flow path F4b, the second central flow path F4a, and the second discharge flow path F4c.

The first central flow path F2a and the second central flow path F4a are arranged so that at least parts of the flow paths overlap each other. The first central flow path F2a and the second central flow path F4a are separated by a porous membrane M. In the chip 1 for cell culture, the first central flow path F2a is a lower layer flow path located in the lower part of the porous membrane M. The second central flow path F4a is an upper flow path located in the upper part of the porous membrane M.

The flow path widths of the first central flow path F2a and the second central flow path F4a can be appropriately set according to the purpose of the chip 1 for cell culture. Examples of the flow path width include 1 $\mu$m to 2,000 $\mu$m, 10 $\mu$m to 1,500 $\mu$m, 50 $\mu$m to 1,000 $\mu$m, and 100 $\mu$m to 500 $\mu$m.

The cover substrate L6 is provided with an opening part W so that the first central flow path F2a and the second central flow path F4a can be observed.

<Resin Included in Flow Path Substrate>

In the chip for cell culture, the flow path substrate (the first flow path substrate L2 and the second flow path substrate L4) includes a resin having a glass transition temperature (hereinafter also referred to as a "Tg") of 37°C or higher, an elastic modulus at 25°C of $1 \times 10^9$ Pa or more, and an elastic modulus at 150°C of $1 \times 10^7$ Pa or less (hereinafter also referred to as a "resin A"). A flow path is formed in the flow path substrate by laser processing. In the chip for cell culture, the flow path substrate forms a partition wall of the flow path.

<<Resin (A)>>

The resin (A) has a Tg of 37°C or higher, an elastic modulus at 25°C of $1 \times 10^9$ Pa or more, and an elastic modulus at 150°C of $1 \times 10^7$ Pa or less.

The "glass transition temperature (Tg)" is a temperature at which glass transition occurs. The Tg can be determined as a temperature at which the tangents intersect with each other at a bending inflection point of the measured temperature obtained by differential scanning calorimetry (DSC) under the condition of a temperature rising rate of 20°C/min.

In the present specification, the "elastic modulus" is a complex elastic modulus measured using a dynamic viscoelasticity measuring device. Specifically, the elastic modulus can be obtained by manufacturing a resin sheet with a test piece having a size of 5 mm $\times$ 40 mm and measuring a dynamic elastic modulus of the test piece using a dynamic viscoelasticity measuring device. As the measurement conditions, conditions in which the temperature is raised from a start temperature of 25°C to 150°C at a temperature rising rate of 2°C/min under a tensile condition of a frequency of 1 Hz may be adopted. As the dynamic viscoelasticity measuring device, for example, Rheogel-E4000 (manufactured by UBM Co., Ltd.) or the like can be used.

The elastic modulus at 25°C is a complex elastic modulus at 25°C, which is measured by the dynamic viscoelasticity measuring device. The elastic modulus at 150°C is a complex elastic modulus at 150°C that is measured by the dynamic viscoelasticity measuring device.

The resin (A) has a Tg of 37°C or higher. A lower limit value of the Tg of the resin (A) is preferably 40°C or higher, more preferably 45°C or higher, still more preferably 50°C or higher, and particularly preferably 55°C or higher, 60°C or higher, or 65°C or higher. An upper limit value of the Tg of the resin (A) is not particularly limited, but is, for example, preferably 110°C or lower, more preferably 100°C or lower, still more preferably 95°C or lower, and particularly preferably 90°C or lower, or 85°C or lower. The Tg of the resin (A) is preferably 40°C to 110°C, more preferably 45°C to 100°C, still more preferably 50°C to 95°C, and particularly preferably 55°C to 90°C, 60°C to 90°C, or 65°C to 85°C. In a case where the Tg of the resin (A) is within the range, the sorption of a drug onto the flow path partition wall is suppressed.

The resin (A) has an elastic modulus of at 25°C of $1 \times 10^9$ Pa or more. The elastic modulus of the resin (A) at 25°C is preferably $1.5 \times 10^9$ Pa or more. The upper limit value of the elastic modulus of the resin (A) at 25°C is not particularly limited, but is, for example, preferably $1 \times 10^{12}$ Pa or less, preferably $1 \times 10^{11}$ Pa or less, more preferably $5 \times 10^{10}$ Pa or less, still more preferably $3 \times 10^{10}$ Pa or less, and particularly preferably $2 \times 10^{10}$ Pa or less. The elastic modulus of the resin (A) at 25°C is preferably $1 \times 10^9$ Pa or more and $1 \times 10^{12}$ Pa or less, more preferably $1 \times 10^9$ Pa or more and $1 \times 10^{11}$ Pa or less, still more preferably $1 \times 10^9$ Pa or more and $5 \times 10^{10}$ Pa or less, and particularly preferably $1 \times 10^9$ Pa or more and $3 \times 10^{10}$ Pa or less. In a case where the elastic modulus at 25°C is within the range, the rigidity of the flow path substrate is maintained.

The resin (A) has an elastic modulus at 150°C of $1 \times 10^7$ Pa or less. The elastic modulus at 150°C of the resin (A) is preferably $8 \times 10^6$ Pa or less, and more preferably $5 \times 10^6$ Pa or less. The lower limit value of the elastic modulus at 150°C of the resin (A) is not particularly limited, but is, for example, preferably $1 \times 10^2$ Pa or more, more preferably $1 \times 10^3$ Pa or more, still more preferably $1 \times 10^4$ Pa or more, even still more preferably $1 \times 10^5$ Pa or more, and particularly preferably $1 \times 10^6$ Pa or more. The elastic modulus at 150°C of the resin (A) is preferably $1 \times 10^2$ Pa or more and $1 \times 10^7$ Pa or less, more preferably $1 \times 10^3$ Pa or more and $1 \times 10^7$ Pa or less, still more preferably $1 \times 10^4$ Pa or more and $1 \times 10^7$ Pa or less, and particularly preferably $1 \times 10^5$ Pa or more and $1 \times 10^7$ Pa or less. In a case where the elastic modulus at 150°C is within the range, the occurrence of irregularities in a processed surface is suppressed at the time of forming a flow path by laser processing.

Examples of the resin (A) include a polyester-based resin, an acrylic resin, a cycloolefin copolymer-based resin, a urethane-based resin, a polyolefin-based resin, a fluorine-based resin, a silicone-based resin, and a mixture or modified resin of these resins. Among those, the resin (A) is preferably the polyester-based resin, the acrylic resin, or the cycloolefin copolymer-based resin.

The "polyester-based resin" refers to a resin including an ester bond (-CO-O-) in the main chain. Examples of the polyester-based resin include a polyester resin and a polyester urethane resin.

The polyester resin can be obtained by copolymerizing a polyvalent carboxylic acid and a polyol. Examples of a commercially available product of the polyester resin as the resin (A) include G7 manufactured by Kurabo Industries Ltd. (Tg: 80°C, elastic modulus at 25°C: $1.84 \times 10^9$ Pa, elastic modulus at 150°C: $3.00 \times 10^5$ Pa), and TP220 (Tg: 70°C, elastic modulus at 25°C: $1.99 \times 10^9$ Pa, elastic modulus at 150°C: $2.74 \times 10^3$ Pa), TP235 (Tg: 25°C, elastic modulus at 65°C: $2.38 \times 10^9$ Pa, elastic modulus at 150°C: $1.42 \times 10^4$ Pa), and TP236 (Tg: 60°C, elastic modulus at 25°C: $2.06 \times 10^9$ Pa, elastic modulus at 150°C: $6.22 \times 10^4$ Pa), all manufactured by Mitsubishi Chemical Corporation.

Examples of the number-average molecular weight (Mn) of the polyester resin as the resin (A) include 5,000 to 100,000. The number-average molecular weight (Mn) is determined based on polystyrene conversion according to gel permeation chromatography (GPC).

The acrylic resin is a polymer of an acrylic acid ester or a methacrylic acid ester. The acrylic resin can be obtained by polymerizing an acrylic acid ester or a methacrylic acid ester. Examples of the acrylic resin as the resin (A) include polymethyl methacrylate (PMMA). Examples of a commercially available product of PMMA include Acrylite™ EX manufactured by Mitsubishi Chemical Corporation (Tg: 70°C, elastic modulus at 25°C: $3.04 \times 10^9$ Pa, elastic modulus at 150°C: $3.51 \times 10^6$ Pa).

Examples of the number-average molecular weight (Mn) of the acrylic resin as the resin (A) include 5,000 to 200,000.

The cycloolefin copolymer-based resin is a copolymer of a cycloolefin and any monomer. The cycloolefin copolymer-based resin can be obtained by copolymerization of a cycloolefin and any monomer. Examples of a commercially available product of the cycloolefin copolymer-based resin as the resin (A) include APL6509T manufactured by Mitsui Chemicals, Inc. (Tg: 80°C, elastic modulus at 25°C: $1.86 \times 10^{10}$ Pa, elastic modulus at 150°C: $1.71 \times 10^6$ Pa).

Examples of the number-average molecular weight (Mn) of the cycloolefin copolymer-based resin as the resin (A) include 5,000 to 150,000.

The resin (A) may be used alone or in combination of two or more kinds thereof.

In a case where the flow path substrate has a multilayer structure, it is preferable that the flow path substrate has at least one layer including the resin (A) (hereinafter also referred to as an "A layer").

The content of the resin (A) in the A layer is preferably 70% by mass or more, more preferably 80% by mass or more, still more preferably 90% by mass or more, and particularly preferably 95% by mass or more with respect to a total mass (100% by mass) of the resin contained in the A layer. The content of the resin (A) in the A layer may be 100% by mass with respect to the total mass (100% by mass) of the resins contained in the A layer.

The A layer may contain a resin other than the resin (A). In addition, the A layer may contain additives such as a solvent (for example, cyclohexanone and propylene glycol-1-methyl ether acetate (PGMEA)), a surfactant, and an antifoaming agent, as appropriate.

The thickness of the A layer is not particularly limited as long as the respective members of the flow path substrate can be adhered. Examples of a lower limit value of the thickness of the A layer include 50 μm or more, 100 μm or more, 150 μm or more, 200 μm or more, 250 μm or more, 300 μm or more, 350 μm or more, 400 μm or more, 450 μm or more, and 500 μm or more. Examples of the upper limit value of the thickness of the A layer include 2,000

μm or less, 1,500 μm or less, 1,000 μm or less, 900 μm or less, 800 μm or less, 750 μm or less, 700 μm or less, and 650 μm or less. Examples of a range of the thickness of the A layer include 50 μm to 2,000 μm, 100 μm to 1,500 μm, 150 μm to 1,000 μm, 200 μm to 900 μm, 250 μm to 800 μm, 300 μm to 750 μm, 350 μm to 700 μm, 400 μm to 650 μm, and 450 μm to 650 μm. The thickness provided as exemplary examples above is the thickness of one A layer.

The flow path substrate may include one or more A layers. Examples of the number of the A layers included in the flow path substrate include 1 to 5, 1 to 4, 1 to 3, and 1 or 2.

A proportion (Ra) of a total thickness (ta) of the A layers to an overall thickness (T1) of the flow path substrates is preferably 50% or more, more preferably 60% or more, still more preferably 70% or more, and particularly preferably 80% or more. An upper limit value of the proportion (Ra) is not particularly limited, and may be 100%. The proportion is preferably 95% or less, more preferably 90% or less, and still more preferably 85% or less. Examples of a range of the proportion include 50% to 100%, 60% to 95%, 65% to 90%, 70% to 90%, and 75% to 85%. In a case where the proportion (Ra) is in the preferred range, the adsorption and the sorption of the drug onto the partition wall of the flow path are suppressed. The proportion (Ra) is determined by Expression (1).

$$Ra\ (\%) = ta/T1 \times 100 \quad (1)$$

< <Resin Having Tg of Lower than 37°C: Resin (B)>>

In a case where the flow path substrate has a multilayer structure, the flow path substrate may include other layers in addition to the A layer. Examples of such other layers include a layer (hereinafter also referred to as a "B layer") containing a resin having a Tg of lower than 37°C (hereinafter also referred to as a "resin (B)"). In a case where the flow path substrate includes the B layer, the adhesion to other members is more easily performed.

Examples of the resin (B) include a polyester-based resin having a Tg of lower than 37°C. Examples of the polyester-based resin include a polyester resin and a polyester urethane resin.

Examples of a commercially available product of the polyester resin having a Tg of lower than 37°C include VYLON 300 (7°C), VYLON 630 (7°C), VYLON 650 (10°C), VYLON GK130 (15°C), VYLON GK140 (20°C), VYLON GK150 (20°C), VYLON GK190 (11°C) VYLON GK330 (16°C), VYLON GK590 (15°C), VYLON GK680 (10°C), VYLON GK780 (36°C), VYLON GK890 (17°C), VYLON 500 (4°C), VYLON 550 (-15°C), and VYLON GK570 (0°C) (all manufactured by Toyobo Co., Ltd.), ELITEL UE-3510 (-25°C), ELITEL UE-3400 (-20°C), ELITEL UE -3220 (5°C), and ELITEL UE-3500 (15°C) (all manufactured by Unitika Ltd.), and G6 (-60°C) (manufactured by Kurabo Industries Ltd.). The numbers in parentheses indicate Tg.

Examples of the number-average molecular weight (Mn) of the polyester resin as the resin (B) include 5,000 to 100,000.

Examples of a commercially available product of the polyester urethane resin having a Tg of lower than 37°C include VYLON UR-8300 (23°C), VYLON UR-3500 (10°C), and VYLON UR-6100 (-30°C). The numbers in parentheses indicate Tg.

Examples of the number-average molecular weight (Mn) of the polyester urethane resin as the resin (B) include 5,000 to 100,000.

The polyester-based resin may be crosslinked with a melamine resin or the like. Examples of the melamine resin include "SUMIMAR (registered trademark) UR" series (manufactured by Sumitomo Chemical Co., Ltd.) and "CYMEL (registered trademark)" series (manufactured by Mitsui Cytec Ltd.).

Furthermore, from the viewpoint of a balance between the workability and the durability, it is preferable that the ratio of the resin to a crosslinking agent is set so that the crosslinking agent (after the reaction) is blended to be in the range of 5% by mass or more and 30% by mass or less in the dried second adhesive layer.

The resin (B) may have an elastic modulus at 150°C of $1 \times 10^7$ Pa or less. The elastic modulus at 150°C of the resin (B) may be, for example, $8 \times 10^6$ Pa or less, or $5 \times 10^6$ Pa or less. A lower limit value of the elastic modulus at 150°C of the resin (B) is not particularly limited, but examples thereof include $1 \times 10^2$ Pa or more, $1 \times 10^3$ Pa or more, $1 \times 10^4$ Pa or more, $1 \times 10^5$ Pa or more, or $1 \times 10^6$ Pa or more. The elastic modulus at 150°C of the resin (B) may be, for example, $1 \times 10^2$ Pa or more and $1 \times 10^7$ Pa or less, $1 \times 10^3$ Pa or more and $1 \times 10^7$ Pa or less, $1 \times 10^4$ Pa or more and $1 \times 10^7$ Pa or less, or $1 \times 10^5$ Pa or more and $1 \times 10^7$ Pa or less.

The resin (B) may have an elastic modulus at 25°C of $1 \times 10^9$ Pa or more, or $1.5 \times 10^9$ Pa or more. An upper limit value of the elastic modulus at 25°C of the resin (B) is not particularly limited, but examples thereof include $1 \times 10^{12}$ Pa or less, $1 \times 10^{11}$ Pa or less, $5 \times 10^{10}$ Pa or less, $3 \times 10^{10}$ Pa or less, or $2 \times 10^{10}$ Pa or less. The elastic modulus at 25°C of the resin (B) may be $1 \times 10^9$ Pa or more and $1 \times 10^{12}$ Pa or less, $1 \times 10^9$ Pa or more and $1 \times 10^{11}$ Pa or less, $1 \times 10^9$ Pa or more and $5 \times 10^{10}$ Pa or less, or $1 \times 10^9$ Pa or more and $3 \times 10^{10}$ Pa or less.

The resin (B) may be a resin other than polyester-based resin. Examples of such another resin include an acrylic resin, a urethane-based resin, a polyolefin-based resin, a fluorine-based resin, a silicone-based resin, and a mixture or modified resin of these resins.

The resin (B) may be used alone or in combination of two or more kinds thereof.

The content of the resin (B) in the B layer is preferably 70% by mass or more, more preferably 80% by mass or more, still more preferably 90% by mass or more, and particularly preferably 95% by mass or more with respect to a total mass (100% by mass) of the resin contained in the B layer. The content of the resin (B) in the second adhesive may be 100% by mass with respect to the total mass (100% by mass) of the resins contained in the second adhesive.

The B layer may contain a resin other than the resin (A) and the resin (B). The B layer may contain additives such as a solvent (for example, cyclohexanone, or propylene glycol-1-methyl ether acetate (PGMEA)), a surfactant, and an antifoaming agent, as appropriate.

In a case where the flow path substrate has the B layer, the thickness of the B layer is preferably 100 μm or less, more preferably 90 μm or less, still more preferably 80 μm or less, and particularly preferably 70 μm or less, or 60 μm or less. Examples of a lower limit value of the thickness of the second adhesive layer include 1 μm or more, 3 μm or more, 5 μm or more, and 7 μm or more. Examples of a range of the thickness of the second adhesive layer include 1 μm to 100 μm, 3 μm to 90 μm, 5 μm to 80 μm, 5 μm to 70 μm, 5 μm to 60 μm, and 7 μm to 60 μm. The thickness provided as exemplary examples above is the thickness of one B layer. In a case where the thickness of the B layer is within the preferred range, the reduction in the drug is likely to be suppressed.

In the chip for cell culture, a proportion (Rb) of a total thickness (tb) of the B layers to an overall thickness (T) of the chip for cell culture is preferably 20% or less. In a case where the proportion (Rb) is set to 20% or less, the reduction in the drug is likely to be suppressed. The proportion (Rb) is preferably 19% or less, more preferably 18% or less, still more preferably 17% or less, and particularly preferably 16% or less, 15% or less, or 14% or less. In a case where the chip for cell culture does not have the second adhesive layer, a proportion of the thickness (tb) of the second adhesive layer is 0%. The proportion (Rb) is determined by Expression (1).

$$Rb\,(\%) = tb/T \times 100 \quad (2)$$

<Configuration Example of Laminate>

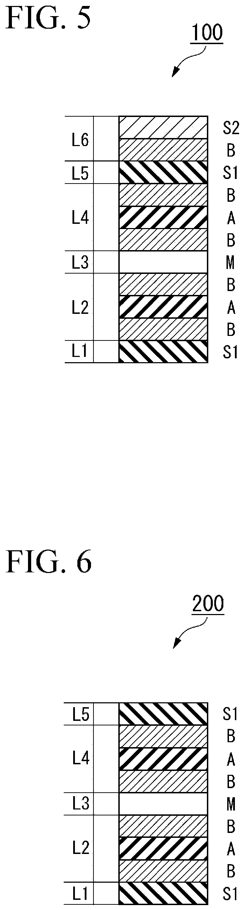

A configuration example of the laminate constituting the chip for cell culture is shown in FIG. 5.

A laminate 100 shown in FIG. 5 is composed of a bottom plate substrate L1, a first flow path substrate L2, a porous membrane L3, a second flow path substrate L4, a top plate substrate L5, and a cover substrate L6 in this order from the bottom. In FIG. 5, A represents the A layer and B represents the B layer.

<<Bottom Plate Substrate L1>>

The bottom plate substrate L1 forms a bottom part of the first flow path F2. In the laminate 100, the bottom plate substrate L1 is composed of an S1 layer. A material of the S1 layer is not particularly limited, but is preferably a material having high biocompatibility. From the viewpoint that observation may be performed using a phase contrast microscope or the like while culturing cells with the chip 1 for cell culture, the S1 layer is preferably made of a having transparency, and more preferably made of a material with low self-fluorescence. In order to enhance the transparency, it is preferable that the S1 layer does not include a filler (anti-blocking agent).

Examples of the transparent material with low self-fluorescence include glass, polyethylene terephthalate (PET), polycarbonate, a cycloolefin polymer, polydimethylsiloxane, polystyrene, and polyacrylate (acryl resin). Among these, PET is preferable as the material of the S1 layer.

The S1 layer may be provided with a lubricative layer including a lubricant component on at least one surface thereof. In addition, a binder resin component may be blended into the lubricative layer.

The lubricant component is not particularly limited, and examples thereof include paraffin wax, microwax, polypropylene wax, polyethylene wax, ethylene-acrylic wax, stearic acid, behenic acid, 12-hydroxystearic acid, stearic acid amide, oleic acid amide, erucic acid amide, methylene bisstearic acid amide, ethylene bisstearic acid amide, ethylene bisoleic acid amide, butyl stearate, stearic acid monoglyceride, pentaerythritol tetrastearate, hydrogenated castor oil, stearyl stearate, siloxane, a higher alcohol-based polymer, stearyl alcohol, calcium stearate, zinc stearate, magnesium stearate, lead stearate, a silicone (dimethylsiloxane)-based low-molecular-weight substance (oil), and a silicone (dimethylsiloxane)-based resin. The lubricant component may be used alone or may be used in a combination of two or more kinds thereof.

Examples of the binder resin component to be blended in the lubricative layer include various resins such as a polyester-based resin, a polyamide-based resin, a polyurethane-based resin, an epoxy-based resin, a phenolic resin, an acrylic resin, a polyvinyl acetate-based resin, a cellulose-based resin, a styrene-based resin, and copolymer resins thereof. From the viewpoint of exhibiting an excellent lubricating property by combining the above-described lubricant component with the binder resin component, a styrene-acrylic copolymer resin is preferable as the binder resin component.

Furthermore, the term "silicone-based" indicates organosiloxanes. The properties thereof may be oily, rubbery, and resinous properties, which are correspondingly referred to as silicone oil, silicone rubber, and a silicone resin in this order. Since any of these have a water-repellent function, a lubrication function, a mold release function, and the like, in a case where the organosiloxanes are contained in the outermost layer portion of a film, the friction of the surface is effectively decreased.

The thickness of the S1 layer is not particularly limited, but can be, for example, 50 $\mu$m to 300 $\mu$m. Examples of the thickness of the substrate S1 include 100 $\mu$m to 250 $\mu$m, and 150 $\mu$m to 200 $\mu$m.

<<First Flow Path Substrate L2>>

A first flow path F2 is formed in the first flow path substrate L2. In the chip for cell culture, the first flow path substrate L2 forms a partition wall of the first flow path F2. In the laminate 100, the first flow path substrate L2 has a multilayer structure.

In the laminate 100, the first flow path substrate L2 is composed of a laminate in which the B layer, the A layer, and the B layer are laminated in this order. By providing the B layer on both surfaces of the A layer, the adhesion between the bottom plate substrate L1 and the porous membrane L3 is easily performed. The B layer functions as an adhesive layer.

The thickness of the first flow path substrate L2 defines a height (a width in the vertical direction) of the first flow path F2. Therefore, the thickness of the first flow path substrate L2 can be appropriately set according to a desired height of the first flow path F2. The thickness of the first flow path substrate L2 may be appropriately adjusted, for example, by modifying the thickness of the A layer.

Examples of the thickness of the first flow path substrate L2 include 300 $\mu$m to 2,000 $\mu$m. Examples of the thickness of the first flow path substrate L2 include 300 $\mu$m to 2,000 $\mu$m, 400 to 1,800 $\mu$m, 500 $\mu$m to 1,500 $\mu$m, 600 $\mu$m to 1,200 $\mu$m, 600 $\mu$m to 1,000 $\mu$m, 600 $\mu$m to 9,000 $\mu$m, 600 $\mu$m to 800 $\mu$m, and 700 $\mu$m to 800 $\mu$m.

<<Porous Membrane L3 > >

The porous membrane L3 partitions the first flow path F2 and the second flow path F4. The porous membrane L3 is composed of a porous membrane M.

The porous membrane M is not particularly limited as long as it has a pore size through which cells to be cultured do not pass and a drug solution can pass. Examples of an average pore size of the porous membrane M include 0.05 $\mu$m to 10 $\mu$m. Examples of a pore density of the porous membrane M include approximately $10^5$ to $10^9$ pores/cm$^2$.

The porous membrane M is preferably composed of a highly biocompatible material. Examples of the material of the porous membrane M include polycarbonate, polyester, polyethylene terephthalate, and polytetrafluoroethylene.

The thickness of the porous membrane M is not particularly limited. Examples of the thickness of the porous membrane M include 0.1 $\mu$m to 100 $\mu$m, 0.5 $\mu$m to 50 $\mu$m, 1 $\mu$m to 30 $\mu$m, 5 $\mu$m to 20 $\mu$m, and 5 $\mu$m to 15 $\mu$m.

<<Second Flow Path Substrate L4>>

A second flow path F4 is formed in the second flow path substrate L4. In the chip for cell culture, the second flow path substrate L4 forms a partition wall of the second flow path F4. In the laminate 100, the second flow path substrate L4 has a multilayer structure.

In the laminate 100, the layer configuration of the second flow path substrate L4 is the same as that of the first flow path substrate L2. That is, it is composed of a laminate in which the B layer, the A layer, and the B layer are laminated in this order. By providing the B layer on both surfaces of the A layer, the adhesion between the porous membrane L3 and the second flow path substrate L4 is more easily performed. The B layer functions as an adhesive layer.

The thickness of the second flow path substrate L4 defines a height (a width in the vertical direction) of the second flow path F4. Therefore, the thickness of the second flow path substrate L4 can be appropriately set according to the height of the target second flow path F4. The thickness of the second flow path substrate L4 may be appropriately adjusted, for example, by modifying the thickness of the A layer.

Examples of the thickness of the second flow path substrate L4 include 300 $\mu$m to 2,000 $\mu$m. Examples of the thickness of the first flow path substrate L2 include 300 $\mu$m to 2,000 $\mu$m, 400 $\mu$m to 1,800 $\mu$m, 500 $\mu$m to 1,500 $\mu$m, 600 $\mu$m to 1,200 $\mu$m, 600 $\mu$m to 1,000 $\mu$m, 600 $\mu$m to 9,000 $\mu$m, 600 $\mu$m to 800 $\mu$m, and 700 $\mu$m to 800 $\mu$m.

<<Top Plate Substrate L5>>

The top plate substrate L5 forms a ceiling of the second flow path F4. In the laminate 100, the top plate substrate L5 is composed of an S1 layer. Examples of the S1 layer include the same layers as those for the S1 layer in the bottom plate substrate L1.

The thickness of the top plate substrate L5 is not particularly limited, but can be, for example, 50 to 600 $\mu$m. Examples of the thickness of the top plate substrate L5 include 100 $\mu$ to 500 $\mu$m, and 150 $\mu$m to 400 $\mu$m.

<<Cover Substrate L6>>

The cover substrate L6 is provided on the uppermost layer of the chip for cell culture. In the laminate 100, the cover substrate L6 is composed of a laminate in which the B layer and the layer S2 are laminated in this order. The B layer functions as an adhesive layer.

From the viewpoint that observation may be performed with a phase contrast microscope or the like while culturing cells with the chip 1 for cell culture, the S2 layer is preferably made of a material having transparency, and more preferably made of a material of low self-fluorescence. In order to enhance the transparency, it is preferable that the S2 layer does not include a filler (anti-blocking agent).

Examples of the transparent material with low self-fluorescence include glass, polyethylene terephthalate (PET), polycarbonate, a cycloolefin polymer, polydimethylsiloxane, polystyrene, polyacrylate (acryl resin), and polymethyl methacrylate (PMMA). Among these, PMMA is preferable as the material of the S2 layer.

The thickness of the S2 layer is not particularly limited, but can be, for example, 500 $\mu$m to 5,000 $\mu$m. Examples of the thickness of the S2 layer include 800 $\mu$m to 3,000 $\mu$m, 1,000 $\mu$m to 2,500 $\mu$m, and 1,500 $\mu$m to 2,500 $\mu$m.

The thickness of the cover substrate L6 is not particularly limited, but can be, for example, 500 $\mu$m to 5,000 $\mu$m. Examples of the thickness of the substrate S2 include 800 $\mu$m to 3,000 $\mu$m, 1,000 $\mu$m to 2,500 $\mu$m, and 1,500 $\mu$m to 2,500 $\mu$m.

A plurality of the S1 layers included in the laminate 100 may all be composed of the same material, or may be partially composed of different materials. The thicknesses of the plurality of the S1 layers may be the same as or different from each other.

A plurality of the A layers included in the laminate 100 may all be composed of layers having the same composition, or may be partially composed of layers having different compositions. The thicknesses of the plurality of the A layers may be the same as or different from each other.

A plurality of the B layers included in the laminate 100 may all be composed of layers having the same composition, or may be partially composed of layers having different compositions. The thicknesses of the plurality of the B layers may be the same as or different from each other.

(Modification Examples)

The number of the A layers included in the first flow path substrate L2 and the second flow path substrate L4 can be appropriately modified. The number of the A layers may be, for example, 1, 2, 4, or 5. In a case where the number of the A layers is increased, the number of the B layers may be increased accordingly.

A part or all of the plurality of the B layers may be eliminated.

An additional flow path substrate may be provided in addition to the first flow path substrate L2 and the second flow path substrate L4. In addition, a bottom plate substrate for the additional flow path, a porous membrane, and a top plate substrate may be provided.

The B layer may have a multilayer structure. In a case where the B layer has a multilayer structure, the respective layers constituting the B layer may each include different kinds of resins (B). In a case where the B layer has the multilayer structure, the number of layers constituting the B layer is not particularly limited, but may be, for example, 2 to 5, 2 to 4, 2 or 3, or 2. In a case where a plurality of the B layers are present, some of the plurality of the B layers may have a multilayer structure, or all of the plurality of the B layers may have a multilayer structure.

<Other Configuration Examples of Laminate>

Another configuration example of the laminate constituting the chip for cell culture is shown in FIG. 6.

A laminate 200 shown in FIG. 6 is composed of a bottom plate substrate L1, a first flow path substrate L2, a porous membrane L3, a second flow path substrate L4, and a top plate substrate L5 in this order from the bottom. In FIG. 6, A is the A layer and B is the B layer.

The laminate 200 is a configuration example that does not include the cover substrate L6. In the laminate 200, the bottom plate substrate L1, the first flow path substrate L2, the porous membrane L3, and the second flow path substrate L4 are the same as those in the laminate 100.

In the laminate 200, the top plate substrate L5 is composed of an S1 layer.

In the laminate 200, the thickness of the top plate substrate L5 is not particularly limited, and examples thereof

include 50 $\mu$m to 300 $\mu$m, 100 $\mu$m to 200 $\mu$m, and 150 $\mu$m to 200 $\mu$m.

The relationship among the plurality of the S1 layers, the plurality of the A layers, and the plurality of the B layers included in the laminate 200 is the same as that in the laminate 100.

According to the chip for cell culture of the present embodiment, the flow path substrate includes a resin A having a glass transition temperature of 37°C or higher, an elastic modulus at 25°C of $1 \times 10^9$ Pa or more, and an elastic modulus at 150°C of $1 \times 10^7$ Pa or less. As a result, in a case where a drug solution is perfused or retained in the flow path, the sorption or adsorption of the drug onto the partition wall of the flow path is suppressed. As a result, a reduction in the drug in the drug solution is suppressed. Accordingly, it is possible to obtain a chip for cell culture in which the reduction in the drug is suppressed.

Since the chip for cell culture of the present embodiment suppresses a reduction in the drug in a case where the drug solution is perfused or retained in the flow path, the chip for cell culture can be suitably used for cell culture in the presence of the drug. The chip for cell culture of the present embodiment can be applied to, for example, an evaluation test (an evaluation test for an efficacy, an evaluation test for a safety, and the like) for a drug using cells.

(Device for Cell Culture)

A second aspect of the present invention is a device for cell culture, including the chip for cell culture of the first aspect.

A device for cell culture is provided with the chip for cell culture of the first aspect, and a mechanism for performing cell culture in the chip for cell culture. The mechanism provided in the device for cell culture can be appropriately modified depending on the purpose of cell culture.

Examples of the mechanism provided in the device for cell culture include a liquid feeding mechanism for supplying a drug solution to a flow path of a chip for cell culture (a drug solution tank, a liquid feeding tube, a delivery pump, or the like), and a drainage mechanism for discharging a drug solution from a flow path of a chip for cell culture (a drainage tank, a drainage tube, a discharge pump, or the like); a temperature maintenance mechanism for maintaining a culture temperature of a chip for cell culture (a thermostat or the like), and a washing mechanism for washing a flow path of a chip for cell culture (a washing liquid tank, a tube, a pump, or the like).

Since the device for cell culture of the present aspect includes the chip for cell culture of the first aspect, cell culture can be performed while suppressing a reduction in a drug in a drug solution. Therefore, the device for cell culture can be suitably used for cell culture in the presence of the drug. The device for cell culture of the present aspect can be applied to, for example, an evaluation test (an evaluation test for an efficacy, an evaluation test for a safety, and the like) for a drug using cells.

(Method for Producing Chip for Cell Culture)

According to a third aspect of the present invention, there is provided a method for producing a chip for cell culture, including a laminate having a flow path structure inside. The production method according to the present aspect includes a step A of laminating a bottom plate substrate, a flow path substrate in which a flow path is formed by laser processing, and a top plate substrate in this order; and a step B of bonding the respective laminated substrates. The flow path substrate includes a resin having a glass transition temperature of 37°C or higher, an elastic modulus at 25°C of $1 \times 10^9$ Pa or more, and an elastic modulus at 150°C of $1 \times 10^7$ Pa or less.

<Step A>

The step A is a step of laminating the bottom plate substrate, the flow path substrate in which a flow path is formed by laser processing, and the top plate substrate in this order.

The flow path substrate may include the first flow path substrate L2 and the second flow path substrate L4. The chip for cell culture may further include a porous membrane L3 and a cover substrate L6.

<<Bottom Plate Substrate L1>>

As the bottom plate substrate L1, the same substrate as described above can be used. The bottom plate substrate L1 can be manufactured by processing the S1 layer into a size of the chip for cell culture, by laser processing or the like. For example, a carbon dioxide laser or the like can be used for the laser processing.

<<First Flow Path Substrate L2>>

As the first flow path substrate L2, the same substrate as described above can be used. The first flow path substrate L2 preferably has a multilayer structure. In a case where the first flow path substrate L2 has the multilayer structure, it is possible to obtain a laminate for the first flow path substrate L2 by manufacturing the respective layers constituting the first flow path substrate L2, and laminating the respective layers, followed by compression-bonding. The compression-bonding of the respective layers can be performed using a laminator, a press machine, or the like. For example, a laminate for the first flow path substrate L2, in which the respective layers are bonded, can be obtained by laminating the respective layers, followed by compression-bonding by a laminator, and then pressing the layers by a press machine.

The laminate for the first flow path substrate L2 can be processed into the size of the chip for cell culture by laser processing. Furthermore, a flow path is formed by laser processing. For example, a carbon dioxide laser or the like can be used for the laser processing.

<<Porous Membrane L3 > >

As the porous membrane L3, the same membrane as described above can be used. The porous membrane L3 can be processed into the size of the chip for cell culture by laser processing or the like. Furthermore, necessary processing such as formation of openings for the port P3 and the port P4 is performed by laser processing or the like.

<<Second Flow Path Substrate L4>>

As the second flow path substrate L4, the same substrate as described above can be used. The second flow path substrate L4 preferably has a multilayer structure. The second flow path substrate L4 can be manufactured by the same method as the first flow path substrate L2.

<<Top Plate Substrate L5>>

As the top plate substrate L5, the same substrate as described above can be used. The top plate substrate L5 may have a multilayer structure or a monolayer structure. In a case where the top plate substrate L5 has the multilayer structure, a laminate for the top plate substrate L5 can be manufactured by the same method as for the first flow path substrate L2.

The laminate for the top plate substrate L5 or the substrate for the top plate substrate L5 can be processed into the size of the chip for cell culture by laser processing or the like. Furthermore, the top plate substrate L5 can be manufactured by performing necessary processing such as formation of openings for the ports P1 to P4 by laser processing or the like.

<<Cover Substrate L6>>

As the cover substrate L6, the same substrate as described above can be used. The cover substrate L6 can be processed into the size of the chip for cell culture by laser processing or the like. Furthermore, the top plate substrate L5 can be manufactured by performing necessary processing such as formation of openings for the ports P1 to P4 and an opening part W by laser processing or the like.

For the respective members prepared as described above, the bottom plate substrate L1, the first flow path substrate L2, the porous membrane L3, the second flow path substrate L4, the top plate substrate L5, and the cover substrate L6 are laminated in this order. At the time of laminating the respective members, the members are laminated so that the positions of the openings for the ports P1 to P4 are aligned with each other. Furthermore, in a case where the chip for cell culture does not include the cover substrate L6, lamination of the cover substrate L6 is not performed.

<Step B>

The step B is a step of bonding the respective laminated substrates.

It is possible to bond the respective substrates using a laminator, a press machine, or the like. For example, by compression-bonding the respective substrates of the laminate with a laminator, followed by pressing with a press machine, it is possible to obtain a chip for cell culture, in which the respective substrates of the laminate are bonded.

The chip for cell culture produced by the production method of the present embodiment is the chip for cell culture of the first aspect. Therefore, the production method according to the present embodiment can be applied to production of the chip for cell culture of the first aspect.

(Method for Culturing Cells)

A fourth aspect of the present invention is a method for culturing cells. The culture method according to the present aspect includes a step of culturing cells in the presence of a drug within the flow path of the chip for cell culture of the first aspect.

<Culturing Step>

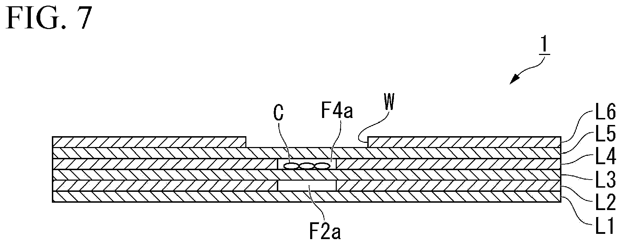

FIG. 7 is a schematic view showing an example of a culture state in the present step. In the chip 1 for cell culture, the first central flow path F2a and the second central flow path F4a are partitioned by the porous membrane L3. Cells C are cultured on the porous membrane L3 facing the second central flow path F4a.

The cells C are not particularly limited and any cell can be used. Animal cells can be used as the cells C. Examples of the animal cells include human cells, and cells of non-human animals (monkeys, mice, rats, guinea pigs, marmosets, dogs, cats, insects, and the like).

The kind of the cells is not particularly limited, and can be appropriately selected according to the intended purpose. Examples of the cells include, but are not limited to, immune cells, germ cells, nerve cells, fibroblasts, mesenchymal stem cells, hormone-secretory cells, cells of various organs, cancer cells, cells of various diseases, and pluripotent stem cells.

Any drug can be used. The drug may be a candidate drug to be developed as a therapeutic agent for any disease. Examples of the drug include, but are not limited to, a low-molecular-weight drug (a molecular weight of 500 or less), a medium-molecular-weight drug (a molecular weight of about 500 to 2,000), and a high-molecular-weight drug (a nucleic acid drug, a protein drug, a polymer, or the like).

The medium that is used in the culture is not particularly limited, and can be appropriately selected according to the kind of the cell. The medium may be a basal medium for animal culture, to which a drug for evaluation and necessary components have been added as appropriate. A known basal medium for animals can be used.

Examples of the basal medium include a Doulbecco's modified Eagle's medium (DMEM), a DMEM/F12 medium, an IMDM medium, a Medium199 medium, an Eagle's minimum essential medium (EMEM), an $\alpha$MEM medium, a Ham's F12 medium, an RPMI 1640 medium, a Fischer's medium, and a mixed medium thereof.

The basal medium may include a serum (such as a fetal bovine serum (FBS)) or a serum replacement, as necessary. Examples of the serum replacement include albumin, transferrin, sodium selenite, ITS-X (Invitrogen), a knockout serum replacement (KSR), an N2 supplement (Invitrogen), a B27 supplement (Invitrogen), a fatty acid, insulin, a collagen precursor, a trace element, 2-mercaptoethanol, and 3'-thiol glycerol. The basal medium may include components such as a lipid, an amino acid, L-glutamine, Glutamax, a non-essential amino acid, a vitamin, a growth factor, an antibiotic, an antioxidant, pyruvic acid, a buffer, and inorganic salts, as necessary. These can be appropriately used in combination.

As the culture conditions, conditions usually used for culturing animal cells can be used. The culture temperature can be, for example, 32°C to 40°C, and is preferably 35°C to 38°C (for example, 37°C). The $CO_2$ concentration can be, for example, about 2% to 5% (for example, 5%).

A culture procedure can be performed, for example, as follows.

First, a medium is injected from the port P1 and introduced into the first central flow path F2a. Next, a cell culture solution is injected from the port P3 and introduced into the second central flow path F4a. Incubation is performed for any time while the cell culture solution is retained in the second central flow path F4a, thereby allowing the cells to be fixed on the porous membrane L3. Next, the drug solution is injected from the port P3 and introduced into the second central flow path F4a, and cell culture is performed. During the cell fixation and the culture, the drug solution may be perfused through the second flow path F4 or may be retained within the second flow path F4. During the culture, the medium may be perfused through the first flow path F2 or may be retained within the first flow path F2.

It is possible to evaluate an effect of the drug on the cells by analyzing the state of the cells during or after the culture.

In the culture method of the present embodiment, since the culture is performed using the chip for cell culture according to the first aspect, it is possible to suppress a reduction in the drug due to sorption and/or adsorption of the chip for cell culture onto the flow path partition wall. Therefore, an effect of the drug on cells can be accurately evaluated.

[Examples]

Hereinafter, the present invention will be described in more detail with reference to Examples, but the present invention is not limited thereto.

(Test on Reduction in Drug)

<Manufacture of Sample for Laser Processing>

A sample of each example shown in Table 1 was cut out by laser processing to manufacture the sample for laser processing. LaserPro (carbon dioxide laser, manufactured by GCC) was used for laser processing of the sample. The output and the speed of the laser were adjusted according to a material of each sample.

[Table 1]

|  | Name of Product | Composition | Name of Manufacturer | Glass transition temperature (°C) |
|---|---|---|---|---|
| Example 1 | KuranBeter G7 | Polyester resin | Kurabo Industries Ltd. | 80 |
| Example 2 | Acrylite™ EX | Polymethyl methacrylate | Mitsubishi Chemical Corporation | 70 |
| Comparative Example 1 | Cosmoshine A4100 | Polyethylene terephthalate | Toyobo Co., Ltd. | 80 |
| Reference Example 1 | VYLON 500 | Polyester resin | Toyobo Co., Ltd. | 4 |
| Reference Example 2 | VYLON GK780 | Polyester resin | Toyobo Co., Ltd. | 36 |

<Measurement of Elastic Modulus>

A test piece (film thickness: 50 $\mu$m, width: 5 mm, and length: 40 mm) for measuring the elastic modulus was cut out from the sample of each example, and the measurement was performed using Rheogel-E4000 (manufactured by UBM Co., Ltd.) under the conditions in which the temperature was raised from a start temperature of 25°C to 150°C at a temperature rising rate of 2°C/min under a tensile condition of a frequency of 1 Hz. The measurement results of the elastic modulus are shown in Table 2.

[Table 2]

|  | Glass transition temperature (°C) | Elastic modulus | |
|---|---|---|---|
|  |  | 25°C | 150°C |
| Example 1 | 80 | 1.84E+09 | 3.00E+05 |
| Example 2 | 70 | 3.04E+09 | 3.51E+06 |
| Comparative Example 1 | 80 | 4.24E+09 | 5.56E+08 |
| Reference Example 1 | 4 | 1.14E+07 | 2.15E+03 |
| Reference Example 2 | 36 | 1.59E+09 | 7.50E+03 |

<Method for Test on Reduction in Drug>

Using a disc sample with the adhesive layer manufactured above, a test on a reduction in five kinds of drugs shown below was performed.

PHN: Phenacetin

DIC: Diclofenac

MEP: (S)-Mephenytoin

BUF: Bufuralol

MDZ: Midazolam

A sample of each example was sterilized by being immersed in 70% ethanol for 30 minutes, and air-dried in a clean bench. The laser-processed sample of Example 2 was sterilized by irradiation with UV (30 minutes per surface) in the clean bench. Next, the sample was placed in a well of a 24-well plate and 550 µL of a drug solution was introduced into the well. Immediately after being introduced into the well, 50 µL was recovered and mixed with 150 µL of a diluent solvent for LC-MS/MS measurement, and the mixture was used as a sample before incubation. Next, the 24-well plate was covered and allowed to stand in an incubator at 37°C. After 48 hours, 50 µL of the drug solution was recovered from the well and mixed with 150 µL of the diluent solvent for LC-MS/MS measurement, and the mixture was used as a sample after 48-hour incubation.

The concentration of the drug in the sample was quantified by LC-MS/MS (EXION AD-QTRAP6500+, manufactured by SCIEX). As a relative value in a case where the concentration of the drug in the sample before incubation was taken as 100%, the residual ratio of the drug of the sample after 48-hour incubation was calculated and is shown in Table 3 as "Residual ratio of drug".

[Table 3]

|  | Laser processing | Residual ratio (%) of drug | | | | |
|---|---|---|---|---|---|---|
|  |  | PHN | DIC | MEP | BUF | MDZ |
| Example 1 | Not performed | 101.1 | 101.6 | 103.9 | 95.3 | 100.3 |
|  | Performed | 94.4 | 97.1 | 97.9 | 73.5 | 88.8 |
| Example 2 | Not performed | 100 | 100 | 100 | 100 | 100 |
|  | Performed | 103.2 | 103.4 | 104.6 | 99 | 99.9 |
| Comparative Example 1 | Not performed | 100 | 100 | 100 | 85 | 100 |
|  | Performed | 93.1 | 95.8 | 97.8 | 45.9 | 80.4 |
| Reference Example 1 | Not performed | 104.9 | 130.9 | 83.8 | 42 | 59.8 |
| Reference Example 2 | Not performed | 79.9 | 90.5 | 79.7 | 42.7 | 65.6 |

From the results, it was confirmed that in Examples 1 and 2, the residual ratio of the drug in the laser-processed sample was not significantly different from the residual ratio of the drug in the laser-unprocessed sample regardless of which drug was used. On the other hand, in Comparative Example 1, the residual ratio of the drug tended to be reduced in the laser-processed sample. Particularly, in MUF, the residual ratio of the drug was greatly reduced in the laser-processed sample.

Reference Examples 1 and 2 are regarding laser-unprocessed samples, and the residual ratio of the drug of some drugs was low.

(Test on Reduction in Drug Using Chip for Cell Culture)

(Manufacture of Chip for Cell Culture)

A chip for cell culture of each example having the following layer configuration was manufactured. The numbers in parentheses indicate the thickness (µm). The resin layers in the respective members are described in order from the lower layer.

[Table 4]

| Member | Example 3 | Example 4 | Example 5 | Comparative Example 2 |
|---|---|---|---|---|
| Bottom plate substrate L1 | S1 (188) | S1 (188) | S1 (188) | S1 (188) |

(continued)

| Member | Example 3 | Example 4 | Example 5 | Comparative Example 2 |
|---|---|---|---|---|
| First flow path substrate L2 | B (60)<br>A1 (600)<br>B (60) | B (60)<br>A1 (2000)<br>B (60) | B (60)<br>A2 (2000)<br>B (60) | B (60)<br>S1 (188)<br>B (60)<br>S1 (188)<br>B (60)<br>S1 (188)<br>B (60) |
| Porous membrane L3 | M (12) | - | - | M (12) |
| Second flow path substrate L4 | B (60)<br>A1 (600)<br>B (60) | - | - | B (60)<br>S1 (188)<br>B (10)<br>S1 (188)<br>B (10)<br>S1 (188)<br>B (60) |
| Top plate substrate L5 | S1 (188) | S1 (188) | S1 (188) | S1 (188) |

S1, A1, A2, B, and M in the layer configuration are as follows.

S1: Cosmoshine A4160 (polyethylene terephthalate)

B: One or two resins including VYLON 500 (polyester resin) (B)

A1: KuranBeter G7 (polyester resin)

RA2: Acrylite™ EX (polymethyl methacrylate)

M: Polyethylene terephthalate-made porous membrane (average pore size: 0.45 $\mu$m, track-etched membrane ip-CELLCUTURE, manufactured by it4ip S. A.)

Sheets for the respective layers of the respective members were manufactured and cut out to a size of 200 mm $\times$ 150 mm. The sheets of the respective layers were laminated according to Table 4 to manufacture a laminate of the respective members. The laminate of the respective members was thermocompression-bonded through a laminate roll (laminator FA-570, manufactured by Taisei Laminator Co., Ltd.) set at 140°C (roll load: 0.3 MPa, speed: 0.1 to 0.5 m/min). Furthermore, the respective layers of the laminate were adhered by pressing at 105°C under a load of 3 t using a small-size press machine (H300-05, manufactured by AS ONE Corporation).

The necessary laser processing was performed for the respective members using LaserPro (carbon dioxide laser, manufactured by GCC). By laser processing, the first flow path substrate L2 was formed of the first flow path F2 and the second flow path substrate L4 was formed of the second flow path F4. Through holes for the port P1 to the port P4 were formed in the top plate substrate L5. Through holes for the port P1 and the port P2 were formed in the porous membrane L3.

A bottom plate substrate L1, a first flow path substrate L2, a porous membrane L3, a second flow path substrate L4, and a top plate substrate L5 are laminated in this order so that positions of the ports and the flow paths are aligned to each other, thereby obtaining a laminate for the chip for cell culture. The laminate was thermocompression-bonded through a laminate roll (laminator FA-570, manufactured by Taisei Laminator Co., Ltd.) set at 140°C (roll load: 0.3 MPa, speed: 0.1 to 0.5 m/min). Furthermore, the respective layers of the laminate were adhered by pressing at 50°C to 80°C under a load of 3 t using a small-size press machine (H300-05, manufactured by AS ONE Corporation).

Then, the resultant was cut out to a size of 58 mm $\times$ 46 mm using LaserPro to obtain a chip for cell culture.

<Method for Test on Reduction in Drug>

Using the chip for cell culture manufactured above, a test on a reduction in a drug shown below was performed.

PHN: Phenacetin
DIC: Diclofenac
MEP: (S)-Mephenytoin
MDZ: Midazolam

The inside of the flow path of the chip for cell culture manufactured above was washed with 9 mL of PBS. Next, the substrate was sterilized by irradiation with UV in a clean bench. UV irradiation was performed for 20 minutes on the front side and the back side of the chip for cell culture. Next, the inside of the flow path was washed with 6 mL of PBS and air-dried in the clean bench.

A drug solution in an amount adjusted to match the volume of the flow path is introduced into the flow path (the first flow path F2, the second flow path F4) of the chip for cell culture, and the chip for cell culture was maintained horizontally so that no air remained within the flow path. 50 μL of the drug solution was recovered immediately after the introduction of the drug solution, and mixed with 150 μL of a diluent solvent for LC-MS/MS measurement, and the mixture was used as a sample after 0-hour incubation.

50 μL of the drug solution was newly introduced into the flow path (the first flow path F2, the second flow path F4) of the chip for cell culture, and left to stand in the incubator at 37°C. After 48 hours from the introduction of the drug solution, 50 μL of the drug solution was recovered and mixed with 150 μL of a diluent solvent for LC-MS/MS measurement, and the mixture was used as a sample after 48-hour incubation.

The concentration of the drug in the sample was quantified by LC-MS/MS. As a relative value in a case where the concentration of the drug of the sample after 0-hour incubation was taken as 100%, the residual ratio of the drug of the sample after 48-hour incubation was calculated and is shown in Table 5 as "Residual ratio of drug".

[Table 5]

|  | Residual ratio (%) of drug | | | |
|---|---|---|---|---|
|  | PHN | DIC | MEP | MDZ |
| Example 3 | 103.4 | 112.5 | 85.1 | 41.1 |
| Example 4 | 97.2 | 105.5 | 97.8 | 52.8 |
| Example 5 | 102.5 | 105.7 | 101.6 | 90.4 |
| Comparative Example 1 | 94.7 | 102.4 | 70.9 | 20.4 |

It was confirmed that the chips for cell culture of Examples can suppress a reduction in the drugs, as compared with the chips for cell culture of Comparative Examples.

Although preferred Examples of the present invention have been described above, the present invention is not limited to these Examples. Addition, omission, replacement, and other modifications can be made to the configuration without departing from the spirit of the present invention. The present invention is not limited by the descriptions above, but only by the scope of the accompanying claims.

[Reference Signs List]

L1: Bottom plate substrate
L2: First flow path substrate
L3: Porous membrane
L4: Second flow path substrate
L5: Top plate substrate
L6: Cover substrate
P1 to P4: Port
F2: First flow path
F4: Second flow path

W: Opening part

**Claims**

1. A chip for cell culture, comprising a laminate having a flow path structure inside,

    wherein the laminate comprises a bottom plate substrate, a flow path substrate in which a flow path is formed by laser processing, and a top plate substrate in this order, and
    the flow path substrate comprises a resin having a glass transition temperature of 37°C or higher, an elastic modulus at 25°C of $1 \times 10^9$ Pa or more, and an elastic modulus at 150°C of $1 \times 10^7$ Pa or less.

2. The chip for cell culture according to Claim 1,

    wherein the flow path substrate comprises a first flow path substrate in which a first flow path is formed and a second flow path substrate in which a second flow path is formed, and
    the laminate further comprises a porous membrane between the first flow path substrate and the second flow path substrate.

3. The chip for cell culture according to Claim 2,
    wherein at least one selected from the group consisting of the first flow path substrate and the second flow path substrate has a multilayer structure.

4. A device for cell culture, comprising the chip for cell culture according to any one of Claims 1 to 3.

5. A method for producing a chip for cell culture, comprising a laminate having a flow path structure inside, the method comprising:

    a step A of laminating a bottom plate substrate, a flow path substrate in which a flow path is formed by laser processing, and a top plate substrate in this order; and
    a step B of bonding the respective laminated substrates,
    wherein the flow path substrate comprises a resin having a glass transition temperature of 37°C or higher, an elastic modulus at 25°C of $1 \times 10^9$ Pa or more, and an elastic modulus at 150°C of $1 \times 10^7$ Pa or less.

6. The method for producing a chip for cell culture according to Claim 5,

    wherein the flow path substrate comprises a first flow path substrate in which a first flow path is formed and a second flow path substrate in which a second flow path is formed, and
    the step A is a step of laminating the bottom plate substrate, the first flow path substrate, a porous membrane, and the top plate substrate in this order through adhesive layers.

7. A method for culturing cells, comprising a step of culturing cells in the presence of a drug within the flow path of the chip for cell culture according to any one of Claims 1 to 3.

FIG. 1

FIG. 2

FIG. 3

_1_

F4a    W

L6
L5
L4
L3
L2
L1

F2a

FIG. 4

_1_

P3        P1

L6
L5
L4
L3
L2
L1

FIG. 5

FIG. 6

FIG. 7

## INTERNATIONAL SEARCH REPORT

International application No.

**PCT/JP2022/032050**

| A. | CLASSIFICATION OF SUBJECT MATTER |
|---|---|

*C12M 1/00*(2006.01)i; *C12N 1/00*(2006.01)i; *B32B 7/02*(2019.01)i; *B32B 7/12*(2006.01)i
FI:   C12M1/00 C; C12N1/00 A; B32B7/12; B32B7/02

According to International Patent Classification (IPC) or to both national classification and IPC

| B. | FIELDS SEARCHED |
|---|---|

Minimum documentation searched (classification system followed by classification symbols)

C12M1/00-3/10; C12N1/00-7/08; B32B7/02; B32B7/12; G01N 33/00-37/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2014-109564 A (NATIONAL INSTITUTE OF ADVANCED INDUSTRIAL & TECHNOLOGY) 12 June 2014 (2014-06-12)<br>paragraphs [0019]-[0025], [0032]-[0034], [0041]-[0052], fig. 1 | 1-7 |
| Y | US 2019/0336973 A1 (THE TRUSTEES OF THE UNIVERSITY OF PENNSYLVANIA) 07 November 2019 (2019-11-07)<br>paragraphs [0003], [0012], [0047], [0048], [0057], [0061]-[0080], fig. 1A-1C | 1-7 |
| Y | Mylar polyester film. Physical-Thermal Properties [online], DuPont Teijin Films, June 2003, [retrieved on 04 October 2022], Internet: <URL:https://usa.dupontteijinfilms.com/wp-content/uploads/2017/01/Mylar_Physical_Properties.pdf><br>table 1, fig. 2 | 1-7 |
| A | JP 2003-33177 A (OKANO, Mitsuo) 04 February 2003 (2003-02-04)<br>entire text | 1-7 |
| A | JP 2021-62417 A (JAPAN ATOMIC ENERGY AGENCY) 22 April 2021 (2021-04-22)<br>entire text | 1-7 |

☑ Further documents are listed in the continuation of Box C.      ☑ See patent family annex.

\*      Special categories of cited documents:
"A"   document defining the general state of the art which is not considered to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search | Date of mailing of the international search report |
|---|---|
| **05 October 2022** | **18 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2022/032050** |

## C.    DOCUMENTS CONSIDERED TO BE RELEVANT

| Category* | Citation of document, with indication, where appropriate, of the relevant passages | Relevant to claim No. |
| --- | --- | --- |
| A | JP 2021-117119 A (SUMITOMO BAKELITE CO., LTD.) 10 August 2021 (2021-08-10)<br>entire text | 1-7 |
| A | JP 2021-114910 A (TOKYO OHKA KOGYO CO., LTD.) 10 August 2021 (2021-08-10)<br>entire text | 1-7 |

Form PCT/ISA/210 (second sheet) (January 2015)

## INTERNATIONAL SEARCH REPORT
### Information on patent family members

International application No.

**PCT/JP2022/032050**

| Patent document cited in search report | | | Publication date (day/month/year) | Patent family member(s) | Publication date (day/month/year) |
|---|---|---|---|---|---|
| JP | 2014-109564 | A | 12 June 2014 | (Family: none) | |
| US | 2019/0336973 | A1 | 07 November 2019 | WO 2015/013364 A1 p. 1, line 14 to p. 2, line 33, p. 6, line 3 to p. 7, line 34, examples, fig. 1a-1c | |
| JP | 2003-33177 | A | 04 February 2003 | (Family: none) | |
| JP | 2021-62417 | A | 22 April 2021 | (Family: none) | |
| JP | 2021-117119 | A | 10 August 2021 | (Family: none) | |
| JP | 2021-114910 | A | 10 August 2021 | US 2021/0222106 A1 entire document EP 3854587 A1 entire document | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021138142 A

- JP 2018102236 A